# EUROPEAN PATENT SPECIFICATION

(11) **EP 1 368 006 B1**
(45) Date of publication and mention of the grant of the patent: **03.05.2006**
(21) Application number: 02710645.9
(22) Date of filing: 08.02.2002
(51) Int. Cl.: A61K 9/22, A61K 9/52, A61K 47/30, A61P 9/08

(54) **NOVEL MODIFIED RELEASED FORMULATION**
NEUE FORMULIERUNG MIT MODIFIZIERTER FREISETZUNG
NOUVELLE FORMULATION A LIBERATION MODIFIEE

(30) Priority: 13.02.2001 SE 0100478; 13.02.2001 SE 0100477
(43) Date of publication of application: 10.12.2003
(73) Proprietor: AstraZeneca AB, 151 85 Södertälje (SE)
(72) Inventor: JUPPO, Anne, S-431 83 Mölndal (SE)
(86) International application number: PCT/SE2002/000228
(87) International publication number: WO 2002/064121

(56) References cited:
- GB-A- 2 160 100
- US-A- 4 629 621
- US-A- 5 405 617
- US-A- 5 965 163

## Description

### Field of the Invention

The present invention is directed to a multiparticulate, modified release solid dispersion formulation comprising a drug substance having a low water-solubility, to a unit dosage of the same, as well as to a process for the preparation thereof. The invention also concerns the use of a multiparticulate, modified release solid dispersion formulation for the manufacture of a medicament for the treatment of various medical conditions such as hypertension.

### Background of the invention

Solubility of a drug in the gastrointestinal fluids and its permeability through the cell membrane determines its oral bioavailability *(Leuner and Dressman, Eur. J. Pharm. Biopharm 50, (2000) 47-60).* For drugs with low aqueous solubility, the dissolution rate in the lumen is the rate-limiting step. Particle size reduction, solubilization, and salt formation are commonly used formulation methods to improve the dissolution rate. However, there are limitations to each of these techniques.

Many drugs do not only have low water solubility, but they might also have a narrow therapeutical index, which means that the drug levels in the blood have to be carefully controlled. This can be achieved by a controlled release formulation. These have other benefits compared to regular dosage forms; patient acceptability is usually better due to fewer doses per day, and the drug is usually more efficiently used so less active drug is needed.

Gel matrix tablets is a common drug form for modified release. The release rate is controlled either by erosion or by the diffusion of drug molecules in the swelled polymer matrix, which is the reason why drug solubility in the matrix material has great influence on the release rate. One disadvantage of matrix tablets is that they cannot always be divided, whereas multiparticulate tablets can be divided.

Solid dispersions have been studied as a possibility to control the drug release rate *(Aceves et al., Int. J. Pharm. 195, (2000) 45-53).* Solid dispersion is a dispersion of one or more active ingredients in an inert carrier or matrix at solid state, prepared by the melting (fusion), solvent or melting-solvent method *(Chiou and Riegelman., J. Pharm. Sci. 60, (1971) 1281-1302).* In *J. Pharm. Sci. 58, (1969) 1505-1509, Chiou and Riegelman* have classified the solid dispersions into following groups: Eutetic mixtures; solid solutions; glass solutions and glass suspensions; amorphous precipitations in crystalline carrier; and combinations of those above.

Melt processing (fusion method) was presented for the first time by *Segikuchi, K. and Obi, N. in 1961, in Chem. Pharm. Bull. 9 (1961), 866-872* to prepare solid dispersions. In the melt method a physical mixture of the carrier and the drug is melted and then solidified. Cooling leads to supersaturation, but due to solidification the dispersed drug is trapped in to the carrier matrix. Melt method is often recommended, because no organic solvents are needed, so it is often less costly and better for the environment than the solvent method. However, it is not a suitable manufacturing method for thermolabile drugs. Thermal degradation, sublimation and polymorphic transformations may also occur during fusion *(Goldberg et al, J. Pharm. Sci.54, (1965) 1145-1148).*

The principle of solid dispersions has been used in many pharmaceutical formulations, mostly in order to increase the bioavailability but in some cases for obtaining sustained release. Solid dispersions can be prepared of lipophilic matrix materials. The release rate is adjusted by varying the drug-excipient ratio. The amount of drug released increases with increased loading *(Bodmeier et al, Drug. Dev. Ind. Pharm. 16 (9), (1990) 1505-1519).*

Besides waxes and polar lipids, different polymers have been used to control drug release rate from solid dispersions. *Ozeki et al.* have shown that the release rate of phenacetin from a solid dispersion composed of poly(ethylene oxide)-carboxyvinylpolymer interpolymer complex can be controlled *(Ozeki et al., J. Control. Release 58, (1999) 87-95).*

US 6, 132, 772 (corresponding to WO 96/23499) discloses an oral, extended release solid pharmaceutical composition comprising polyethylene glycol having a molecular weight of at least 1000, a drug having a solubility of less than 0.1 % by weight in water at 20°C and a hydrophilic gel-forming polymer having a mean molecular weight of at least 20 000.

US 5,965,163 discloses a solid dosage form comprising a plurality of particles. The drug may according to this document be soluble or water insoluble.

US 5,405,617 discloses the preparation of carrier matrices and spray congealed powders comprising an admixture of aliphatic or fatty acid esters and pharmaceutical actives which can be compressed into tablet and caplet dosage form.

US 4,629,621 discloses a sustained release preparation of bioactive material having erodible characteristics.

Stearic acid has been used as a controlled release matrix excipient in spray congealing *(Rodriguez et al., Int. J. Pharm. 183, (1999) 133-143).* The drug substances used by Rodriguez are theophylline having a water solubility at 25°C of 8.3 mg/ml, and fenbufen having a water solubility at 25 °C of 0.11 mg/ml.

### Outline of the invention

The object of the present invention is to provide a pharmaceutical formulation of a drug substance having low solubility in water.

More particularly, the present invention is directed to a multiparticulate, modified release solid dispersion formulation, comprising
(i) an active drug substance having a water-solubility of, or below, 8 mg/ml at room temperature;
(ii) at least one hydrophobic matrix former which is a meltable, non-swelling amphiphilic lipid having a water-solubility below 1 mg/g; and
(iii) at least one hydrophilic matrix former which is a meltable excipient having a water-solubility above 0.1 g/g;
wherein
the weight ratio hydrophobic matrix former/ hydrophilic matrix former is ≥ 1; and the particle size is less than 300 µm.

The term "modified release" is herein defined as a formulation that releases less than 90% of its drug contents during the first three hours of the release.

The wording "at least one hydrophobic matrix former" as used herein, is defined such that one hydrophobic matrix former can be used alone, or in an alternative embodiment of the invention a mixture of hydrophobic matrix formers may be used.

The wording "at least one hydrophilic matrix former" as used herein, is defined such that one hydrophilic matrix former can be used alone, or in an alternative embodiment of the invention a mixture of hydrophilic matrix formers may be used.

The term "solid dispersion" is herein defined as a dispersion of the active compound in an inert carrier or matrix at solid state. Solid dispersion is more particularly defined herein as eutetic mixtures, solid solutions, glass solutions or glass suspensions, amorphous precipitations in crystalline carrier or combinations thereof.

The wording "low solubility in water" used herein, is defined as a substance which at room temperature, such as at a temperature of 23°C, has a solubility in water of, or below, 8 mg/ml.

The wording "multiparticulate formulation" used in accordance with the present invention is defined as a formulation comprising individual units of the drug substance, the hydrophobic matrix former and the hydrophilic matrix former, filled into capsules or compressed into e.g. one single tablet which may be a rapidly disintegrating tablet.

The hydrophobic matrix formers are in accordance with the present invention water-insoluble, non-swelling fatty acids having a melting point above 50°C, more particularly a melting point within the range of from 55-75 °C. Examples of specific fatty acids useful in accordance with the present invention are stearic acid, palmitic acid and myristic acid, or mixtures thereof.

In a further aspect of the invention the hydrophobic matrix former is a fatty acid ester such as, but not limited to, glyceryl monostearate, glyceryl behenate, glyceryl dipalmitostearate, and glyceryl di/tristearate, or mixtures thereof.

In still a further aspect of the invention the hydrophobic matrix former is a hydrogenated fatty acid ester such as, but not limited to, hydrogenated castor oil, also known under the trade mark Cutina HR®.

In still a further aspect of the invention the hydrophobic matrix former is a mixture of mono-, di- and triglycerides and polyethyleneglycol mono- and diesters of fatty acids, such as Gelucire® 50/02.

The hydrophobic matrix former may also be selected from waxes such as carnauba wax; fatty alcohols such as, but not limited to, cetyl alcohol, stearyl alcohol or cetostearyl alcohol, or mixtures thereof.

The hydrophilic matrix formers are in accordance with the present invention meltable, water soluble excipients which are solid at room temperature, such as polyethyleneoxides; polyethylene glycols; and polyethyleneoxide and polypropyleneoxide block-co-polymers, e.g. poloxamers. Specific examples of poloxamers useful in accordance with the present invention are poloxamer 188, also known under the trade name Pluronic F68®, and poloxamer 407, which is also known under the trade name Pluronic F127®. Pluronic F68® and Pluronic F127® are commercially available from BASF. Specific examples of polyethylene glycols useful in accordance with the present invention are PEG 4000, known under the trade name Macrogol 4000®, and PEG 6000, known under the trade name Macrogol 6000®. Any poloxamer and PEG which are solid at room temperature may be used in accordance with the present invention. A comprehensive list of poloxamers and PEG's useful in accordance with the present invention can be found in *Handbook of Pharmaceutical Excipients 3rd Ed., American Pharmaceutical Association and Pharmaceutical Press (2000), Washington, 665,* which is hereby incorporated by reference, but which list however should not in any way be interpreted as exhaustive. Also other hydrophilic excipients which are miscible with the hydrophobic matrix formers as melts are useful in accordance with the present invention. Also other hydrophilic excipients which are miscible with the hydrophobic matrix formers as melts are useful in accordance with the present invention.

The weight ratio of hydrophobic matrix former/ hydrophilic matrix former is ≥ 1, the excess amount of the hydrophobic matrix providing a modified release effect.

In one aspect of the invention, felodipine which has the chemical name 2,6-dimethyl-4-(2,3-dichlorophenyl)-1,4-dihydropyridine-3,5-dicarboxylic acid-3-methyl ester-5-ethyl ester, is used as the active drug substance. Felodipine is an antihypertensive substance disclosed in EP 0 007 293, having a water-solubility of about 0.5 µg/ml at an ambient temperature of 22-25 °C.

A further aspect of the invention is to use bicalutamide, a non-steroidal anti-androgen which is the racemate of 4'-cyano-α',α',α'-trifluoro-3-(4-fluorophenylsulphonyl)-2-hydroxy-2-methylpropiono-*m*-toluidide, as the active drug substance. Bicalutamide is known under the trade name CASODEX™ . Bicalutamide is useful in prostate cancer therapy, and EP 100172 discloses 4'-cyano-α',α',α'-trifluoro-3-(4-fluorophenylsulphonyl)-2-hydroxy-2-methylpropiono-m-toluidide (named in EP 100172 as 4-cyano-3-trifluoromethyl-*N*-(3-*p*-fluorophenylsulphonyl-2-hydroxy-2-methylpropionyl)aniline).4'-cyano-α',α',α'-trifluoro-3-(4-fluorophenylsulphonyl)-2-hydroxy-2-methylpropiono-m-toluidide as well as the racemate thereof, as well as where >50% of the 4'-cyano-α',α',α'-trifluoro-3-(4-fluorophenylsulphonyl)-2-hydroxy-2-methylpropiono-m-toluidide is provided in the form of the R-enantiomer, is also within the scope of the invention. The water-solubility for bicalutamide is about 0.0046 mg/ml at physiological pH and at an ambient temperature of 22-25 °C.

In one embodiment of the invention, the total amount of the active drug substance is below about 40% by weight. In a further aspect of the invention the total amount of the drug substance is 30-40% by weight, and in still a further embodiment of the invention the total amount of the active drug substance is 20-30% by weight.

The wording "unit dosage form" is herein defined as a composition where the amount of active drug substance is administered as one single tablet, capsule or other suitable form in accordance with the present invention.

The pharmaceutical formulation according to the present invention is useful for the treatment of various medical conditions such as cardiovascular diseases or in the treatment of cancer, e.g. prostate cancer.

Thus, one aspect of the present invention is the use of a multiparticulate, modified release formulation as claimed and described herein, for the manufacture of a medicament for the treatment of hypertension or cancer such as prostate cancer.

Another aspect of the present invention, is a method for the treatment of hypertension or cancer such as prostate cancer, whereby a multiparticulate, modified release formulation as claimed and described herein, is administered to a patient in need of such treatment.

The multiparticulate, modified release formulation according to the present invention may be formulated into a unit dosage form, preferably as a tablet or a capsule, which may also comprise standard excipients known to the skilled person in the art of formulation. Examples of such excipients are fillers, binders, disintegrants and lubricants, but this list should however not be interpreted as being exhaustive.

The multiparticulate, modified release solid dispersion formulation according to the present invention provides the possibility of formulating drug substances having a water-solubility of, or below, 8 mg/ml at room temperature. The novel formulation is particularly useful when formulated into a tablet. The multiparticulate system makes it possible to divide the tablet without disturbing the release rate of the active drug substance.

### Methods of preparation

In spray congealing, or spray chilling as it is also called, the melted mass is atomized into droplets, which solidify quickly in cool air *(Killeen, Pharm. Eng., July*/*August 1993, 56-64*). The process differs from spray drying in that in spray drying the main action is evaporation of solvent caused by warm air, whereas in spray congealing it is a phase change from liquid to solid.

The spray congealing process used in accordance with the present invention comprises the following steps:
(i) melting the hydrophobic matrix former;
(ii) dissolving or emulsifying the active compound into the melt;
(iii) dissolving the hydrophilic matrix former into the melt;
(iv) atomizing the melt into droplets;
(v) solidifying the droplets; and
(vi) collecting the particles.

The produced particles can then be further formulated and into tablets or filled into capsules.

The atomization into droplets can be done with different techniques, such as with a capillary nozzle, with a pneumatic nozzle, with an ultrasonic nozzle, with a hydraulic nozzle, with electrospraying, with rotary atomization, and preferably with a pneumatic nozzle using warm air as atomization gas.

The solidification of droplets can take place in liquid nitrogen, in or on carbondioxide ice or in air with a temperature lower than the melt point of the droplets. The particles may be collected into a vessel directly, or with a cylinder connected to a cyclone. The resulted particles are smaller than 300 µm, preferably spherical, and the drug is present in the particles in the form of a solid dispersion.

Additives may be added into the melt prior to the atomization. Examples of such additives are surface active agents, excipients increasing viscosity, and buffering agents, but this list should however not in any way be interpreted as limiting the invention.

Information on the particle size distribution and on the roundness of the particles may be obtained by image analysis system (BeadCheck 300/MC, PharmaVision AB, Lund, Sweden). The particles are distributed on a glass plate with a sample preparation device. The number of particles from each batch are photographed to analyze number size distribution and roundness distribution.

Mean diameter is used for particle size distribution. The radius from the center of mass to the particle perimeter is measured in incremental steps of 3° (BeadCheck™ 830 User's Manual). The diameter of each particle is calculated from the mean value of these measurements.

Roundness is a measurement of the length-width relationship, with a value in the range [0.0, 1.0] (BeadCheck™ 830 Configuration Manual). A perfect circle has roundness 1.0 and a very narrow object has roundness close to 0.

### Detailed description of the invention

The invention will now be described in more detail by way of the following examples, which however should not be construed as limiting the invention in any way.

The following multiparticulate, modified release solid dispersion formulations were prepared. For each of these Examples, the number of 5000 particles (Examples 1-7) or 10 000 particles (Examples 8-11) from each batch were photographed to analyze number size distribution and roundness distribution.

| **Example 1** | amount [g] |
|---|---|
| (i) felodipine | 1 |
| (ii) cetanol | 4 |
| (iii) PEG 4000 | 2 |

### I. Preparation of the multiparticulate, modified release formulation

Felodipine (1 g) was dissolved in a melt of 4 g cetanol at 110°C. The amount of 2 g PEG 4000 was added into the melt. The melted mixture was kept at 110°C and atomized with a pneumatic nozzle by using an atomization air temperature of 400°C and a pressure of 7 bar. The particles were collected into a vessel which was kept on carbondioxide ice (temperature -50°C), and thereafter dried over night in a vacuum oven at 25°C and 2 mbar.

The resulted particles had a 90% fractile size (90% smaller than) of 78 µm and roundness of 0.85.

| **Example 2** | amount [g] |
|---|---|
| (i) felodipine | 1 |
| (ii) cetanol | 4 |
| (iii) poloxamer 407 | 2 |

### I. Preparation of the multiparticulate, modified release formulation

Felodipine (1 g) was dissolved in a melt of 4 g cetanol at 110°C. The amount of 2 g poloxamer 407 (Pluronic F127®) was added into the melt. The melted mixture was kept at 110°C and atomized with a pneumatic nozzle by using an atomization air temperature of 400°C and a pressure of 7 bar. The particles were collected into a vessel which was kept on carbondioxide ice (temperature -50°C), and thereafter dried over night in a vacuum oven at 25°C and-2 mbar.

The resulted particles had a 90% fractile size (90% smaller than) of 77 µm and a roundness of 0.87.

| **Example 3** | amount [g] |
|---|---|
| (i) felodipine | 1 |
| (ii) hydrogenated castor oil | 4 |
| (iii) PEG 4000 | 2 |

### I. Preparation of the multiparticulate, modified release formulation

Felodipine (1 g) was dissolved in a melt of 4 g hydrogenated castor oil (Cutina HR®) at 110°C. The amount of 2 g PEG 4000 was added into the melt. The melted mixture was kept at 110°C and atomized with a pneumatic nozzle by using an atomization air temperature of 400°C and a pressure of 7 bar. The particles were collected into a vessel which was kept on carbondioxide ice (temperature -50°C), and thereafter dried over night in a vacuum oven at 25°C and 2 mbar.

The resulted particles had a 90% fractile size (90% smaller than) of 73 µm and a roundness of 0.90.

| **Example 4** | amount [g] |
|---|---|
| (i) felodipine | 1 |
| (ii) hydrogenated castor oil | 4 |
| (iii) poloxamer 407 | 2 |

### I. Preparation of the multiparticulate, modified release formulation

Felodipine (1 g) was dissolved in a melt of 4 g hydrogenated castor oil (Cutina HR®) at 110°C. The amount of 2 g poloxamer 407 (Pluronic F127®) was added into the melt. The melted mixture was kept at 110°C and atomized with a pneumatic nozzle by using an atomization air temperature of 400°C and a pressure of 7 bar. The particles were collected into a vessel which was kept on carbondioxide ice (temperature -50°C), and thereafter dried over night in a vacuum oven at 25°C and 2 mbar.

The resulted particles had a 90% fractile size (90% smaller than) of 69 µm and a roundness of 0.92.

| **Example 5** | amount [g] |
|---|---|
| (i) felodipine | 1 |
| (ii) glyceryl palmitostearate | 4 |
| (iii) poloxamer 407 | 2 |

### I. Preparation of the multiparticulate, modified release formulation

Felodipine (1 g) was dissolved in a melt of 4 g glyceryl palmitostearate (Precirol® ATO 5) at 110°C. The amount of 2 g poloxamer 407 (Pluronic F127®) was added into the melt. The melted mixture was kept at 110°C and atomized with a pneumatic nozzle by using an atomization air temperature of 400°C and a pressure of 7 bar. The particles were collected into a vessel which was kept on carbondioxide ice (temperature -50°C), and thereafter dried over night in a vacuum oven at 25°C and 2 mbar.

The resulted particles had a 90% fractile size (90% smaller than) of 72 µm and a roundness of 0.94.

| **Example 6** | amount [g] |
|---|---|
| (i) felodipine | 1 |
| (ii) Stearic acid | 4 |
| (iii) PEG 4000 | 2 |

### I. Preparation of the multiparticulate, modified release formulation

Felodipine (1 g) was dissolved in a melt of 4 g stearic acid at 110°C. The amount of 2 g PEG 4000 was added into the melt. The melted mixture was kept at 110°C and atomized with a pneumatic nozzle by using an atomization air temperature of 400°C and a pressure of 7 bar. The particles were collected into a vessel which was kept on carbondioxide ice (temperature -50°C), and thereafter dried over night in a vacuum oven at 25°C and 2 mbar.

The resulted particles had a 90% fractile size (90% smaller than) of 77 µm and roundness of 0.93.

| **Example 7** | amount [g] |
|---|---|
| (i) felodipine | 1 |
| (ii) Stearic acid | 4 |
| (iii) poloxamer 407 | 2 |

### I. Preparation of the multiparticulate, modified release formulation

Felodipine (1 g) was dissolved in a melt of 4 g stearic acid at 110°C. The amount of 2 g poloxamer 407 (Pluronic F127®) was added into the melt. The melted mixture was kept at 110°C and atomized with a pneumatic nozzle by using an atomization air temperature of 400°C and a pressure of 7 bar. The particles were collected into a vessel which was kept on carbondioxide ice (temperature -50°C), and thereafter dried over night in a vacuum oven at 25°C and 2 mbar.

The resulted particles had a 90% fractile size (90% smaller than) of 70 µm and a roundness of 0.94.

| **Example 8** | amount [g] |
|---|---|
| (i) felodipine | 2 |
| (ii) stearic acid | 6 |
| (iii) poloxamer 407 | 6 |

Felodipine (2 g) was dissolved in a melt of 6 g stearic acid at 1 10°C. The amount of 6 g poloxamer 407 (Pluronic F127®) was added into the melt. The melted mixture was kept at 110°C and atomised with a pneumatic nozzle by using an atomisation air temperature of 400°C and a pressure of 7 bar. The particles were collected into a vessel which was kept on carbondioxide ice (temperature -50°C), and thereafter dried over night in a vacuum oven at 25°C and 2 mbar.

The resulted particles had a 90% fractile size (90% smaller than) of 56 µm and roundness of 0.96.

| **Example 9** | amount[g] |
|---|---|
| (i) felodipine | 2 |
| (ii) glyceryl ditristearate | 8 |
| (iii) poloxamer 407 | 4 |

Felodipine (2 g) was dissolved in a melt of 8 g glyceryl ditristearate (Precirol WL2155®) at 110°C. The amount of 4 g poloxamer 407 (Pluronic F127®) was added into the melt. The melted mixture was kept at 110°C and atomised with a pneumatic nozzle by using an atomisation air temperature of 400°C and a pressure of 7 bar. The particles were collected into a vessel which was kept on carbondioxide ice (temperature -50°C), and thereafter dried over night in a vacuum oven at 25°C and 2 mbar.

The resulted particles had a 90% fractile size (90% smaller than) of 49 µm and roundness of 0.93.

| **Example 10** | amount [g] |
|---|---|
| (i) felodipine | 2 |
| (ii) glyceryl behenate | 8 |
| (iii) poloxamer 407 | 4 |

Felodipine (2 g) was dissolved in a melt of 8 g glyceryl behenate (Compritol 888®) at 1 10°C.The amount of 4 g poloxamer 407 (Pluronic F127®) was added into the melt. The melted mixture was kept at 110°C and atomised with a pneumatic nozzle by using an atomisation air temperature of 400°C and a pressure of 7 bar. The particles were collected into a vessel which was kept on carbondioxide ice (temperature -50°C), and thereafter dried over night in a vacuum oven at 25°C and 2 mbar.

The resulted particles had a 90% fractile size (90% smaller than) of 51 µm and roundness of 0.97.

| **Example 11** | amount[g] |
|---|---|
| (i) felodipine | 2 |
| (ii) glyceryl monostearate | 8 |
| (iii) poloxamer 407. | 4 |

Felodipine (2 g) was dissolved in a melt of 8 g glyceryl monostearate at 110°C.The amount of 4 g poloxamer 407 (Pluronic F127®) was added into the melt. The melted mixture was kept at 110°C and atomised with a pneumatic nozzle by using an atomisation air temperature of 400°C and a pressure of 7 bar. The particles were collected into a vessel which was kept on carbondioxide ice (temperature -50°C), and thereafter dried over night in a vacuum oven at 25°C and 2 mbar.

The resulted particles had a 90% fractile size (90% smaller than) of 50 µm and roundness of 0.99.

### II. Tabletting

Particles from step I of each of the examples 1-11 above, were compressed into tablets, which had a theoretical felodipine content of 10 mg. The target tablet weight was 200 mg. Tablet mass consisted of 35% particles and 65% microcrystalline cellulose. The mixture of microparticles, microcrystalline cellulose and sodium stearyl fumarate (0.14% of the total mixture weight) was mixed in a Turbula mixer of the type 72C, Willy A. Bachofen AG Maschinenfabrik, Basle, Switzerland, for 10 minutes. This mixture was compressed with an excentric tablet press Kilian SP300 (Examples 1-7) or Kilian EK0 (Examples 8-11) using 10.0 mm flat punches with maximum compression forces of 5.0-5.6 kN (Examples 1-7) or 2.7-7.0 kN (Examples 8-11).

The breaking force of resulting tablets was within the range 43-93 N.

### III. Dissolution tests of tablets

The rate of release was tested from all tablet samples from examples using USP II paddle method. Dissolution test from each batch was run three times. Release testing was performed in a dissolution medium of 500 ml of sodium dihydrogen phosphate buffer at pH 6.5. 0.4 % cetyl trimethylammonium bromide was added to the buffer to increase the solubility of felodipine. The measurements were carried out at 37°C and the paddle was rotated 100 rpm. Each tablet was placed in a basket located about 1 cm above the paddle. Aliquots (10 ml) were withdrawn after 0.5, 1, 2, 4, and 7 hours and filtered through 1.2 µm filter (Millipore® MF-MilIipore). The first 5 ml of the filtrate was discarded.

The filtrated sample solutions were then analyzed with UV-spectrophotometer at wavelength 362 nm and 450 nm.

The results of the dissolution for each Example above, are summarized in Table 1 below.

**Table 1**

| **Example No.** | **% Dissolved in 4 hours** | **% Dissolved in 7 hours** |
|---|---|---|
| **Reference Example:** | | |
| A standard tablet comprising: | 88 | 95 |
| (i) 10 mg felodipine; and | | |
| (ii) 190 mg microcrystalline cellulose | | |
| (Avicel PH101®) | | |
| 1 | 12 | 18 |
| 2 | 29 | 41 |
| 3 | 39 | 51 |
| 4 | 50 | 61 |
| 5 | 45 | 89 |
| 6 | 13 | 18 |
| 7 | 16 | 26 |
| 8 | 62 | 92 |
| 9 | 57 | 82 |
| 10 | 54 | 65 |
| 11 | 68 | 91 |

## Claims

1. A multiparticulate, modified release solid dispersion formulation, comprising
(i) an active drug substance having a water-solubility of, or below, 8 mg/ml at room temperature;
(ii) at least one hydrophobic matrix former which is a meltable, non-swelling amphiphilic lipid having a water-solubility below 1 mg/g; and
(iii) at least one hydrophilic matrix former which is a meltable excipient having a water-solubility above 0.1 g/g;
wherein
the weight ratio hydrophobic matrix former/ hydrophilic matrix former is ≥ 1; and the particle size is less than 300 µm.

2. A multiparticulate, modified release solid dispersion formulation according to claim 1, wherein the hydrophobic matrix former or mixture thereof, is a water-insoluble, non-swelling fatty acid having a melting point above 50 °C.

3. A multiparticulate, modified release solid dispersion formulation according to claim 2, wherein the hydrophobic matrix former or mixture thereof, is a water-insoluble, non-swelling fatty acid having a melting point of from 55-75°C.

4. A multiparticulate, modified release solid dispersion formulation according to any one of the preceding claims, wherein the hydrophobic matrix former or mixture thereof, is selected from any one of stearic acid, palmitic acid and myristic acid.

5. A multiparticulate, modified release solid dispersion formulation according to claim 1, wherein the hydrophobic matrix former is a fatty acid ester.

6. A multiparticulate, modified release solid dispersion formulation according to claim 5, wherein the hydrophobic matrix former or mixture thereof, is selected from any one of glyceryl monostearate, glyceryl behenate, glyceryl dipalmitostearate, and glyceryl di/tristearate.

7. A multiparticulate, modified release solid dispersion formulation according to claim 1, wherein the hydrophobic matrix former is a hydrogenated fatty acid ester.

8. A multiparticulate, modified release solid dispersion formulation according to claim 7, wherein the hydrophobic matrix former is hydrogenated castor oil.

9. A multiparticulate, modified release solid dispersion formulation according to claim 1, wherein the hydrophobic matrix former is a mixture of mono-, di- and triglycerides and polyethyleneglycol esters of fatty acids.

10. A multiparticulate, modified release solid dispersion formulation according to claim 1, wherein the hydrophobic matrix former is selected from waxes, fatty alcohols or mixtures thereof.

11. A multiparticulate, modified release solid dispersion formulation according to claim 10, wherein the hydrophobic matrix former is carnauba wax.

12. A multiparticulate, modified release solid dispersion formulation according to claim 10, wherein the hydrophobic matrix former is selected from any one of cetyl alcohol, stearyl alcohol and cetostearyl alcohol, or mixtures thereof.

13. A multiparticulate, modified release solid dispersion formulation according to any one of claims 1-12, wherein the hydrophilic matrix former is selected from any one of polyethyleneoxides, polyethyleneglycols, polyethyleneoxide and polypropyleneoxide block-co-polymers, or mixtures thereof.

14. A multiparticulate, modified release solid dispersion formulation according to claim 13, wherein the hydrophilic matrix former is a poloxamer.

15. A multiparticulate, modified release solid dispersion formulation according to claim 14, wherein the poloxamer is poloxamer 407.

16. A multiparticulate, modified release solid dispersion formulation according to claim 13, wherein the hydrophilic matrix former is a polyethylene glycol.

17. A multiparticulate, modified release solid dispersion formulation according to claim 16, wherein the hydrophilic matrix former is PEG 4000 or PEG 6000.

18. A multiparticulate, modified release solid dispersion formulation according to any one of the previous claims, wherein the active drug substance is felodipine or bicalutamide.

19. A multiparticulate, modified release solid dispersion formulation according to any one of the previous claims, wherein the total amount of the drug substance is below about 40 % by weight.

20. A unit dosage form comprising a multiparticulate, modified release solid dispersion formulation according to any one of claims 1-19.

21. A tablet comprising a multiparticulate, modified release solid dispersion formulation according to any one of claims 1-19, further comprising one or more pharmaceutically acceptable excipients.

22. A tablet according to claim 21, wherein the pharmaceutically acceptable excipients are microcrystalline cellulose and sodium stearyl fumarate.

23. A process for the preparation of a multiparticulate, modified release formulation according to any one of claims 1-19, whereby said formulation is prepared by spray congealing.

24. A process according to claim 23, whereby the spray congealing comprises the following steps
(i) melting the hydrophobic matrix former;
(ii) dissolving or emulsifying the active compound into the melt;
(iii) dissolving the hydrophilic matrix former into the melt;
(iv) atomizing the melt into droplets;
(v) solidifying the droplets; and
(vi) collecting the particles.

25. Use of a multiparticulate, modified release solid dispersion formulation according to any one of claims 1-19, for the manufacture of a medicament for the treatment of a cardiovascular disease.

26. Use of a multiparticulate, modified release solid dispersion formulation according to any one of claims 1-19, for the manufacture of a medicament for use in cancer therapy.

## Patentansprüche

1. Multipartikuläre feste Dispersionsformulierung mit modifizierter Freisetzung, enthaltend
(i) einen Arzneimittelwirkstoff mit einer Löslichkeit in Wasser von 8 mg/ml oder weniger bei Raumtemperatur;
(ii) wenigstens einen hydrophoben Matrixbildner, bei dem es sich um ein schmelzbares, nicht aufquellendes amphiphiles Lipid mit einer Löslichkeit in Wasser von unter 1 mg/g handelt; und
(iii) wenigstens einen hydrophilen Matrixbildner, bei dem es sich um einen schmelzbaren Hilfsstoff mit einer Löslichkeit in Wasser von mehr als 0,1 g/g handelt;
wobei
das Gewichtsverhältnis von hydrophobem Matrixbildner zu hydrophilem Matrixbildner ≥ 1 ist und
die Teilchengröße unter 300 µm liegt.

2. Multipartikuläre feste Dispersionsformulierung mit modifizierter Freisetzung nach Anspruch 1, wobei es sich bei dem hydrophoben Matrixbildner bzw. einer Mischung davon um eine wasserunlösliche, nicht aufquellende Fettsäure mit einem Schmelzpunkt über 50°C handelt.

3. Multipartikuläre feste Dispersionsformulierung mit modifizierter Freisetzung nach Anspruch 2, wobei es sich bei dem hydrophoben Matrixbildner bzw. einer Mischung davon um eine wasserunlösliche, nicht aufquellende Fettsäure mit einem Schmelzpunkt von 55-75°C handelt.

4. Multipartikuläre feste Dispersionsformulierung mit modifizierter Freisetzung nach einem der vorhergehenden Ansprüche, wobei der hydrophobe Matrixbildner bzw. die Mischung davon ausgewählt ist aus Stearinsäure, Palmitinsäure und Myristinsäure.

5. Multipartikuläre feste Dispersionsformulierung mit modifizierter Freisetzung nach Anspruch 1, wobei es sich bei dem hydrophoben Matrixbildner um einen Fettsäureester handelt.

6. Multipartikuläre feste Dispersionsformulierung mit modifizierter Freisetzung nach Anspruch 5, wobei der hydrophobe Matrixbildner bzw. eine Mischung davon ausgewählt ist aus Glycerylmonostearat, Glycerylbehenat, Glyceryldipalmitostearat und Glyceryldi/tristearat.

7. Multipartikuläre feste Dispersionsformulierung mit modifizierter Freisetzung nach Anspruch 1, wobei es sich bei dem hydrophoben Matrixbildner um einen hydrierten Fettsäureester handelt.

8. Multipartikuläre feste Dispersionsformulierung mit modifizierter Freisetzung nach Anspruch 7, wobei es sich bei dem hydrophoben Matrixbildner um hydriertes Ricinusöl handelt.

9. Multipartikuläre feste Dispersionsformulierung mit modifizierter Freisetzung nach Anspruch 1, wobei es sich bei dem hydrophoben Matrixbildner um eine Mischung von Mono-, Di- und Triglyceriden und Polyethylenglykolestern von Fettsäuren handelt.

10. Multipartikuläre feste Dispersionsformulierung mit modifizierter Freisetzung nach Anspruch 1, wobei der hydrophobe Matrixbildner ausgewählt ist aus Wachsen, Fettalkoholen oder Mischungen davon.

11. Multipartikuläre feste Dispersionsformulierung mit modifizierter Freisetzung nach Anspruch 10, wobei es sich bei dem hydrophoben Matrixbildner um Carnaubawachs handelt.

12. Multipartikuläre feste Dispersionsformulierung mit modifizierter Freisetzung nach Anspruch 10, wobei der hydrophobe Matrixbildner ausgewählt ist aus Cetylalkohol, Stearylalkohol und Cetostearylalkohol oder Mischungen davon.

13. Multipartikuläre feste Dispersionsformulierung mit modifizierter Freisetzung nach einem der Ansprüche 1-12, wobei der hydrophile Matrixbildner ausgewählt ist aus Polyethylenoxiden, Polyethylenglykolen, Polyethylenoxid- und Polypropylenoxid-Blockcopolymeren oder Mischungen davon.

14. Multipartikuläre feste Dispersionsformulierung mit modifizierter Freisetzung nach Anspruch 13, wobei es sich bei dem hydrophilen Matrixbildner um ein Poloxamer handelt.

15. Multipartikuläre feste Dispersionsformulierung mit modifizierter Freisetzung nach Anspruch 14, wobei es sich bei dem Poloxamer um poloxamer 407 handelt.

16. Multipartikuläre feste Dispersionsformulierung mit modifizierter Freisetzung nach Anspruch 13, wobei es sich bei dem hydrophilen Matrixbildner um ein Polyethylenglykol handelt.

17. Multipartikuläre feste Dispersionsformulierung mit modifizierter Freisetzung nach Anspruch 16, wobei es sich bei dem hydrophilen Matrixbildner um PEG 4000 oder PEG 6000 handelt.

18. Multipartikuläre feste Dispersionsformulierung mit modifizierter Freisetzung nach einem der vorhergehenden Ansprüche, wobei es sich bei dem Arzneimittelwirkstoff um Felodipin oder Bicalutamid handelt.

19. Multipartikuläre feste Dispersionsformulierung mit modifizierter Freisetzung nach einem der vorhergehenden Ansprüche, wobei die Gesamtmenge an Arzneimittel unter etwa 40 Gew.-% liegt.

20. Einheitsdosisform, enthaltend eine multipartikuläre feste Dispersionsformulierung mit modifizierter Freisetzung nach einem der Ansprüche 1-19.

21. Tablette, enthaltend eine multipartikuläre feste Dispersionsformulierung mit modifizierter Freisetzung nach einem der Ansprüche 1-19, weiterhin enthaltend einen oder mehrere pharmazeutisch annehmbare Hilfsstoffe.

22. Tablette nach Anspruch 21, wobei es sich bei den pharmazeutisch annehmbaren Hilfsstoffen um mikrokristalline Cellulose und Natriumstearylfumarat handelt.

23. Verfahren zur Herstellung einer multipartikulären Formulierung mit modifizierter Freisetzung nach einem der Ansprüche 1-19, wobei die Formulierung durch Sprüherstarren hergestellt wird.

24. Verfahren nach Anspruch 23, wobei das Sprüherstarren die folgenden Schritte beinhaltet:
(i) Schmelzen des hydrophoben Matrixbildners;
(ii) Lösen oder Emulgieren des Wirkstoffs in der Schmelze;
(iii) Lösen des hydrophilen Matrixbildners in der Schmelze;
(iv) Zerstäuben der Schmelze zu Tröpfchen;
(v) Erstarren der Tröpfchen und
(vi) Sammeln der Partikel.

25. Verwendung einer multipartikulären festen Dispersionsformulierung mit modifizierter Freisetzung nach einem der Ansprüche 1-19 zur Herstellung eines Medikaments zur Behandlung einer Herzkreislauferkrankung.

26. Verwendung einer multipartikulären festen Dispersionsformulierung mit modifizierter Freisetzung nach einem der Ansprüche 1-19 zur Herstellung eines Medikaments zur Verwendung in der Krebstherapie.

## Revendications

1. Formulation multiparticulaire de dispersion de solides à libération modifiée, comprenant
(i) une substance médicamenteuse active ayant une solubilité dans l'eau d'au plus 8 mg/ml à la température ambiante;
(ii) au moins une matière hydrophobe formant une matrice qui est un lipide amphiphile fusible, non gonflant, ayant une solubilité dans l'eau inférieure à 1 mg/g; et
(iii) au moins une matière hydrophile formant une matrice qui est un excipient fusible ayant une solubilité dans l'eau supérieure à 0,1 g/g;
dans laquelle
le rapport pondéral entre matière hydrophobe formant une matrice et la matière hydrophile formant une matrice est ≥ 1; et
la granulométrie est inférieure à 300 µm.

2. Formulation multiparticulaire de dispersion de solides à libération modifiée selon la revendication 1, dans laquelle la matière hydrophobe formant une matrice, ou un mélange d'entre elles, est un acide gras non gonflant insoluble dans l'eau ayant un point de fusion supérieur à 50°C.

3. Formulation multiparticulaire de dispersion de solides à libération modifiée selon la revendication 2, dans laquelle la matière hydrophobe formant une matrice, ou un mélange d'entre elles, est un acide gras non gonflant insoluble dans l'eau ayant un point de fusion qui va de 55-75°C.

4. Formulation multiparticulaire de dispersion de solides à libération modifiée selon l'une quelconque des revendications précédentes, dans laquelle la matière hydrophobe formant une matrice, ou un mélange d'entre elles, est choisie indifféremment parmi l'acide stéarique, l'acide palmitique et l'acide myristique.

5. Formulation multiparticulaire de dispersion de solides à libération modifiée selon la revendication 1, dans laquelle la matière hydrophobe formant une matrice est un ester d'acide gras.

6. Formulation multiparticulaire de dispersion de solides à libération modifiée selon la revendication 5, dans laquelle la matière hydrophobe formant une matrice, ou un mélange d'entre elles, est choisie indifféremment parmi le monostéarate de glycéryle, le béhénate de glycéryle, le dipalmitostéarate de glycéryle et le di/tristéarate de glycéryle.

7. Formulation multiparticulaire de dispersion de solides à libération modifiée selon la revendication 1, dans laquelle la matière hydrophobe formant une matrice est un ester d'acide gras hydrogéné.

8. Formulation multiparticulaire de dispersion de solides à libération modifiée selon la revendication 7, dans laquelle la matière hydrophobe formant une matrice est de l'huile de ricin hydrogénée.

9. Formulation multiparticulaire de dispersion de solides à libération modifiée selon la revendication 1, dans laquelle la matière hydrophobe formant une matrice est un mélange de mono-, de di- et de triglycérides et d'esters d'acides gras de polyéthylèneglycol.

10. Formulation multiparticulaire de dispersion de solides à libération modifiée selon la revendication 1, dans laquelle la matière hydrophobe formant une matrice est choisie parmi les cires, les alcools gras ou leurs mélanges.

11. Formulation multiparticulaire de dispersion de solides à libération modifiée selon la revendication 10, dans laquelle la matière hydrophobe formant une matrice est de la cire de carnauba.

12. Formulation multiparticulaire de dispersion de solides à libération modifiée selon la revendication 10, dans laquelle la matière hydrophobe formant une matrice est choisie indifféremment parmi l'alcool cétylique, l'alcool stéarylique et l'alcool cétostéarylique, ou leurs mélanges.

13. Formulation multiparticulaire de dispersion de solides à libération modifiée selon l'une quelconque des revendications 1-12, dans laquelle la matière hydrophile formant une matrice est choisie indifféremment parmi les poly(oxydes d'éthylène), les polyéthylèneglycols, les copolymères séquencés de poly(oxyde d'éthylène) et de poly(oxyde de propylène), ou leurs mélanges.

14. Formulation multiparticulaire de dispersion de solides à libération modifiée selon la revendication 13, dans laquelle la matière hydrophile formant une matrice est un poloxamère.

15. Formulation multiparticulaire de dispersion de solides à libération modifiée selon la revendication 14, dans laquelle le poloxamère est le poloxamère 407.

16. Formulation multiparticulaire de dispersion de solides à libération modifiée selon la revendication 13, dans laquelle la matière hydrophile formant une matrice est un polyéthylèneglycol.

17. Formulation multiparticulaire de dispersion de solides à libération modifiée selon la revendication 16, dans laquelle la matière hydrophile formant une matrice est le PEG 4000 ou le PEG 6000.

18. Formulation multiparticulaire de dispersion de solides à libération modifiée selon l'une quelconque des revendications précédentes, dans laquelle la substance médicamenteuse active est la félodipine ou le bicalutamide.

19. Formulation multiparticulaire de dispersion de solides à libération modifiée selon l'une quelconque des revendications précédentes, dans laquelle la quantité totale de la substance médicamenteuse est inférieure à environ 40% en poids.

20. Forme posologique unitaire comprenant une formulation multiparticulaire de dispersion de solides à libération modifiée selon l'une quelconque des revendications 1-19.

21. Comprimé comprenant une formulation multiparticulaire de dispersion de solides à libération modifiée selon l'une quelconque des revendications 1-19, et comprenant en outre un ou plusieurs excipients pharmaceutiquement acceptables.

22. Comprimé selon la revendication 21, dans lequel les excipients pharmaceutiquement acceptables sont la cellulose microcristalline et le stéarylfumarate de sodium.

23. Procédé de préparation d'une formulation multiparticulaire à libération modifiée selon l'une quelconque des revendications 1-19, selon lequel ladite formulation est préparée par congélation par atomisation.

24. Procédé selon la revendication 23, selon lequel la congélation par atomisation comprend les étapes suivantes :
(i) faire fondre la matière hydrophobe formant une matrice;
(ii) dissoudre ou émulsionner le composé actif dans la masse fondue;
(iii) dissoudre la matière hydrophile formant une matrice dans la masse fondue;
(iv) atomiser la masse fondue en gouttelettes;
(v) solidifier les gouttelettes; et
(vi) recueillir les particules.

25. Utilisation d'une formulation multiparticulaire de dispersion de solides à libération modifiée selon l'une quelconque des revendications 1-19, pour fabriquer un médicament destiné au traitement d'une maladie cardiovasculaire.

26. Utilisation d'une formulation multiparticulaire de dispersion de solides à libération modifiée selon l'une quelconque des revendications 1-19, pour fabriquer un médicament à utiliser dans le traitement du cancer.
